# EUROPEAN PATENT APPLICATION

(11) **EP 2 078 960 A1**
(43) Date of publication of application: **15.07.2009**
(21) Application number: 08000456.7
(22) Date of filing: 11.01.2008
(51) Int. Cl.: G01N 33/68, G01N 33/53

(54) **Method for determining the type of allergy**

(71) Applicant: BIOMAY AG, 1090 Wien (AT)
(72) Inventor: Valenta, Rudolf, 2604 Theresienfeld (AT); Campana, Raffaela, 1140 Vienna (AT); Mothes-Luksch, Nadine, Dr., 1180 Vienna (AT)
(74) Representative: Keller, Günter

(57) **Abstract**

The present application discloses an in vitro method for determining the type of allergy wherein a sample
a) is reacted with an allergen comprising at least one epitope to which IgE antibodies bind and
b) the sample is further reacted with a hypoallergen derivative essentially corresponding to the allergen as used in step a) which does not contain any IgE epitope and
c) the extent of the reaction between allergen and the sample and the corresponding hypoallergen and the sample is measured.

## Description

A high percentage of the population in particular in industrialized countries suffers from allergy. Allergy against plant pollen, mites, molds and food becomes a severe problem for patients suffering from this disease. There are different forms of allergy. Some patients suffer immediately after contact with the allergen under varying degrees of severity. Others suffer chronic manifestations of the contact with the allergen.

The IgE antibodies play an important role in allergic reactions. It is becoming increasingly evident that IgE antibodies can have diverse pathological effects. It is well-established that IgE antibodies bind tightly by means of the high affinity receptor FcεRI to mast cells and basophils. Crosslinking of receptors by allergen-bound IgE induces the release of inflammatory mediators like histamine and leukotrienes which cause the immediate symptoms of the disease. Such symptoms may be rhinitis, conjunctivitiy and asthma. This type of allergic reaction is called type I reaction whereby IgE antibodies, T_{H}2-cells and mast cells are involved.

Very frequently, however, late reactions following the contact of the patient with the allergen can be observed. Such late reactions are caused by the presentation of allergens to T-cells which become activated, proliferate and release proinflammatory cytokines like for example interleukins such as IL-4, IL-5, IL-6 and IL-13, interferon gamma or TNFα. Usually the allergen is presented on an allergen-presenting cell like a B cell, a macrophage or a dendritic cell via the receptor FcεRI or RcεRII, respectively, and the antibody bound thereto. The antigen-presenting cells bind to a T_{H}2 cell which proliferates and secretes the cytokines, for example interleukin-5 which induces tissue eosinophils and the release of inflammatory mediators from eosinophils. In order to select suitable therapeutic measures it is helpful to know from which type of allergic disease the patient is suffering.

It is therefore an object of the present invention to provide a diagnostic method which allows the determination of the type of allergy. In a preferred embodiment the diagnostic method is performed in vitro.

This determination of the type of allergy is in particular relevant when the allergic individual suffers from chronic manifestations of atopy like for example chronic asthma and/or atopic dermatitis and/or further to rhinitis and/or allergy against food.

The present invention provides therefore in vitro method for determining the type of allergy wherein a sample
a) is reacted with an allergen comprising at least one epitope to which IgE antibodies bind and
b) the sample is further reacted with a hypoallergen derivative corresponding to the allergen as used in step a) which does not contain any IgE epitope and
c) the extent of the reaction between allergen and the sample and the corresponding hypoallergen and the sample is measured.

The method of the present invention is an in vitro method which means that the diagnostic method will not be performed on the body of the subject to be tested. Preferably the sample to be tested is obtained from the blood of the patient, preferably it is a serum sample or a preparation of peripheral blood mononuclear cells (PBMCs) or target organ-derived cell samples. In the method two tests are performed in parallel with an aliquot of the sample whereby one test is performed with an allergen which comprises at least one epitope to which IgE antibodies can bind. The other aliquot is tested with a hypoallergen derivative of the allergen which is nearly identical with the allergen except that the hypoallergen does not contain any IgE epitope.

The testing is preferably performed by measuring and comparing IgE-mediated effects with the IgE-epitope-bearing allergens with non-IgE-mediated effects using a hypoallergen devoid of IgE epitopes.

Both the allergen and the hypoallergen are subjected to each of the tests procedures in order to ensure the lack of IgE reactivity of the hypoallergen.

### Measurements for IgE-mediated effects can be:

Simple IgE reactivity can be measured whereby the sample is serum, plasma, secretions (nasal fluids, tears, bronchial lavages etc).

In another embodiment the sample are IgE-bearing cells such as mast cells, basophils, B cells, eosinophils, antigen-presenting cells etc. In such embodiments activation can be determined by measurement of released mediators, proteases, cytokines, measurement of the up or down-regulation of certain surface markers, activation of other cells by antigen-presenting cells in the form of proliferative responses that rely on IgE- facilitated antigen presentation.

In a further embodiment the sample may be an explanted material or a biopsy containing IgE or IgE-bearing cells whose activation can be measured.

Alternatively the sample is tissue which is studied by in vivo exposure.

### Measurements for non-IgE-mediated effects can be:

1. Measurements of T cell activation such as proliferative responses, release of cytokines, up or downregulation of surface markers in peripheral blood samples, or using T cell lines or T cell clones.

It is also possible to measure the tissue-damaging properties of the substances released from the cells which are activated by non-IgE-mediated effects by transferring the supernatants from the activated cells onto other cells or tissues and studying the toxic, damaging or proapoptotic effects of the products from cells which have been activated in an non-IgE-dependent manner onto other cells or tissues for example as described in Mittermann et al., J. Invest. Dermatol. (2007), vol. 00, pp 1-8.

### 2. Measurement of non-IgE-mediated activation in biopsies.

### 3. Measurement of non-IgE-mediated effects in vivo using e.g., the atopy patch test

Whenever there is a positive reaction with the hypoallergen lacking IgE epitopes, there is evidence for non-IgE-mediated tissue damage e.g., by T cells.

Any or several of the methods for measuring IgE-mediated effects can be combined with one or several of the methods for measuring non-IgE-mediated effects in a test kit.

In a preferred embodiment of the present invention the diagnostic method is performed with peripheral blood mononuclear cells (PBMCs) which are obtained from the patient to be tested. Peripheral blood mononuclear cells are blood cells having a round nucleus such as a lymphocyte or a monocyte. These blood cells are a critical component of the immune system to fight infection and adapt to intruders and they play an important role in allergy. Such cells can be extracted from whole blood using Ficoll which is a hydrophylic polysaccharide that separates layers of blood with monocytes and lymphocytes forming a buffy coat under a layer of plasma. This buffy coat contains the PBMCs.

After having obtained the PBMCs from the patients, several samples are prepared whereby each contains a nearly identical number of PBMCs. At least two samples of PBMCs are prepared. One of the samples is brought into contact with the antigen containing the IgE epitope or the IgE epitopes and the other sample is brought into contact with the corresponding hypoallergen derivative which corresponds essentially with the IgE containing epitope with the proviso that the the hypoallergen derivative does not contain any IgE epitope.

After having brought the antigen containing the IgE epitope(s) and the corresponding hypoallergen with the PBMCs in contact proliferative and/or cytokine responses are measured. It can be tested by methods well-known in the art whether the supernatants contain toxic factors and/or cytokines like interferon gamma, tumor nekrosis factor α and the like.

In one preferred embodiment of the present invention the supernatants are brought into contact with respiratory epithelial cell layers and it is determined whether the epithelial cells go to apoptosis. It is evident for the skilled person in the art to prepare the samples in such a manner that equal amount of cells and antigen are used in the test in order to compare the values obtained in the test performed with the allergen containing the IgE epitope(s) and the test performed with the corresponding hypoallergen. The interaction of different cells and apoptosis in atopic dermatitis is described in Akdis et al., Acta Odontol. Scand. (2001), pp 178-182 which is incorporated herewith by reference.

Human bronchial epithelial cell layers which can be used in a preferred embodiment of the present invention can be prepared according to the method as described by Reisinger et al., Journal Allergy Clin. Immunol. (May 2005), pp 973-981. Since the T_{H1}-derived cytokine interferon gamma can lead to decreased epithelial barrier function and increased allergen influx, this embodiment leads to results which resemble as close as possible the allergic situation in nature.

An example of a pair of allergen and hypoallergen is the Bet v 1 allergen as described in Vrtala et al., J.Clin.Invest. 1997, pp 1673-1681.

Other pairs of allergen and corresponding hypoallergen have been reviewed in Valenta R. Nature Reviews Immunology, June 2002, pp 446-452; in Linhart & Valenta Curr. Opin. Immunol. (2005), pp 646-655. Further hypoallergens are described by Vrtala et al., J. Immunol. (2007), pp 1730-1739 (Phl p 6); Westritschnig et al., J. Immunol. (2007), pp 7624-7634 (Phl p 12); Westritschnig et al., J. Immunol. (2004), pp 5684-5692 (Phl p 7).

IgE test methods are described in Wiele, Bennick and Johansson Lancet (1967), pp 1105-1107 describing the RAST; IgE ELISAs are described in Stern et al., J.Allergy Clin. Immunol. (2007), pp 351-358; or Hiller et al., FASEB J (2002), published online January 14, 2002 (chip). The disclosure of the cited publications is incorporated herewith by reference. By using the methods as disclosed in the articles also other pairs of allergen and corresponding hypoallergen can be constructed.

The person skilled in the art can easily detect whether an allergen comprises epitopes to which specifically IgE antibodies bind by reacting the antigen with sera obtained from allergic patients. The binding of IgE antibodies to this allergen is preferably detected with the help of specific anti-IgE-antibodies which can be visualized by appropriate chemical reactions or dyes. Suitable methods are described for example in Vrtala et al., J.Clin.Invest. (April 1997), pp 1673-1681.

The extent of the reaction between the allergen and the corresponding hypoallergen and the serum samples is measured by methods known to the person skilled in the art. It is important that both reactions, namely with the allergen on the one hand and the corresponding hypoallergen on the other hand are performed under comparable conditions which has to be verified by the use of suitable control experiments.

In preferred embodiments of the present invention the sample is obtained from a patient suffering from a chronic allergic disease.

Preferably the serum sample is obtained from a patient who suffers from a chronic allergic disease like for example atopic dermatitis, asthma or other chronic diseases (e.g. chronic rhinitis, allergy against food like allergy against peanuts, celery, certain types of fish or crustaceans). The method of the present invention can also be applied in patients suffering from asthmatic diseases, in particular asthma, allergy against house dust which is caused by mites and/or molds and in patients which are allergic against pets, in particular cats.

The method of the present invention can be preferably used when the allergic disease is an allergic reaction of the late type. In an especially preferred embodiment the allergic disease is atopic dermatitis.

Atopic dermatitis is a chronic inflammatory skin disorder affecting about 3-10% of patients with IgE-mediated allergies. The cardinal features of atopic dermatitis are eczematous and erythematous skin lesions, which are characterized by spongiosis, epidermal hyperplasia and perivascular infiltrates consisting of T-cells, monocytes, macrophages and antigen-presenting cells. Thus, atopic dermatitis resembles many features of delayed-type hypersensitivity. The symptoms of atopic dermatitis are triggered in the vast majority of patients with atopic dermatitis by exogenous exposure to allergens, among them food and aeroallergens. The external application of allergens to the skin by means of atopy patch tests (APTs) can also be be used to elicit the characteristic eczematous skin lesions in patients with atopic dermatitis for diagnostic purposes.

It is an object of the present invention to determine whether the chronic inflammation in such patients is caused by IgE-mediated or non-IgE-mediated mechanisms. Patients suffering from atopic dermatitis with high levels of IgE express high levels of the receptor FcεRI on monocytes and dendritic cells and it has been demonstrated in vitro that IgE-facilitated antigen presentation activates allergen-specific T-cells more efficiently than allergen presentation through non-IgE-mediated mechanisms.

The discrimination between IgE-mediated and non-IgE-mediated mechanisms is important since this may have an important influence on the decision which therapy is selected. Patients suffering from IgE-mediated atopic dermatitis may be treated successfully with neutralizing anti-human IgE antibodies which mostly belong to the IgG class.

On the other hand patients suffering from atopic dermatitis which is non-IgE-mediated can be treated by using therapy strategies targeting T-cells in a relatively selective manner. Such treatment may include the application of cyclosporine or tacrolimos. Other T cell targeting approaches are anti-CD4 antibodies, steroids and the blockade of co-stimulation and biologics which target T cells.

In the vitro methods of the present invention the reaction between allergen and serum sample and corresponding hypoallergen and serum sample is preferably measured in an immunological test.

In the immunological tests according to the present invention the binding of IgE antibodies to the allergen and the corresponding hypoallergen having no IgE epitope is performed by conventional immunological methods whereby IgE antibodies bound to the allergen can be detected by the reactions known to the person skilled in the art like for example colour reactions. On the other hand it may also be advantageous to measure the binding of other antibody types than IgE antibodies like IgG to allergen and hypoallergen, respectively.

In preferred embodiments the methods use immunological tests, in particular an enzyme-linked immunosorbent assay, and/or a dot-blot test.

In an also preferred embodiment the in vitro methods of the present invention measure the reaction between allergen and serum sample and corresponding hypoallergen and serum sample in a histamine release assay.

IgE testing can also be done by RAST, Cap or chip-based assays.

The activation of effector cells can be measured by upregulation of surface markers by FACS such as CD203c or CD63, release of leukotrienes as described in reviews about basophile and mast cell activation tests (see Kleine-Tebbe Int. Arch. Allergy Immunol. (2007), pp 79-90; Hauswirth J. Allergy Clin. Immunol. vol. 110 (2002), pp 102-109, for CD203c test). As references for measuring T cell activation the following publications can be mentioned: Reisinger et al., J. Allergy Clin. Immunol. (2005), pp 973-981 describing the release of IFN-gamma or Mittermann et al., Journal of Investigative Dermatology (2007), pp 1-9.

The invention relates also to test kits for determining the type of allergy which comprises an allergen comprising at least one epitope to which IgE antibodies can bind and a corresponding hypoallergen derivative which does not contain any IgE epitope.

The present invention provides also suitable test kits for determining the type of allergy which comprise at least an allergen and the corresponding hypoallergen derivative in a suitable test kit. The tests can also be performed in skin prick tests. In one embodiment the invention relates therefore also to test kits which are suitable for performing skin prick tests.

Skin prick tests are widely used in allergy testing and can be performed with solutions, preferably aqueous solutions of the allergen. The test kits of the present invention always comprise at least two test solutions wherein in one test solution the allergen having at least one IgE antigen is present whereas in the second test solution the corresponding hypoallergen is present which is free of any IgE epitope. Furthermore, a test kit comprises controls using the diluent which serve as negative control and as positive control preferably a histamine solution. For performing a skin prick test a drop of an allergen solution and a drop of the corresponding hypoallergic allergen-containing solution is placed on the skin of the forearm of the patient. A sterile lancet or a needle is used to prick the skin through the allergen solution whereby a separate needle or lancet is used for each allergen solution. The excess allergen solution is removed from the skin with an absorbent paper tissue. The reaction is evaluated after 15 minutes. It is important that the test is performed with standardized allergen solutions which means that the content of allergen and corresponding hypoallergen are essentially the same.

A positive result is a skin wheel with a diameter greater than 2 mm than that observed with the negative control.

The present invention is further illustrated by the following examples:

### Example 1

To study the contribution of IgE-mediated versus non-IgE-mediated mechanisms in chronic allergic skin inflammation, a purified IgE-reactive major allergen (ie, the birch pollen allergen Bet v 1 [molecular weight, 17.4 kd] and 2 non-IgE-reactive hypoallergenic fragments thereof (F1: amino acids 1-74 [molecular weight, 8 kd]: F2: amino acids 75-160 [molecular weight, 9.4 kd) was used which together comprise the full T-cell epitope repertoire of the Bet v 1 allergen but lack IgE reactivity and allergenic activity (ie, basophile activation and induction of immediate-type skin reactions) for APTs in patients with AD. The used antigen is described in Breiteneder et al., EMBO J [1989], pp. 1935-1938 and Vrtala et al., J.Clin.Invest [1997], pp. 1673-1681, the disclosure of which is enclosed by reference. Skin prick tests and APTs were performed in 5 patients with AD and birch pollen allergy, a patient with birch pollen allergy without skin manifestations, an allergic person without birch pollen allergy, and 2 non-allergic individuals using rBet v 1 or a mix of the 2 rBet v 1 fragments (F1: amino acids 1-74; F2: amino acids 75-160).

**The demographic, clinical, and serologic characterization of the individuals is summarized in Table I.**

| | | | | | | | | | | **SPT (mm²)** | | | | | **APT** | |
|---|---|---|---|---|---|---|---|---|---|---|---|---|---|---|---|---|
| | | | | | **IgE CAP (kUA/I)** | | **IgE** | | | **rBet v 1** | | **F1+F2** | | **Hist.** | | |
| **Patients** | **Sex/Age** | **Allergies** | **Symptoms** | **Total IgE (kU/I)** | **Birch** | **rBet v 1** | **rBet v 1** | **F1** | **F2** | **20 µg/ml** | **40 µg/ml** | **20 µg/ml** | **40 µg/ml** | | **rBet v 1** | **F1+F2** |
| A | M/53 | b, a, g, pf, npf, mo, mi | AD, RC, AS, OAS | 860 | 15.7 | 14.6 | + | - | - | 100 | 132 | - | - | 16.6 | +++ | - |
| B | M/41 | b, pf | RC, AD | 69.3 | 27.4 | 27.1 | + | - | - | 56 | 121 | - | - | 8 | ++ | +++ |
| C | M/45 | b, a, g, pf, mi | RC, AD, OAS | 114 | 25.6 | 20.2 | + | - | - | 156 | 210 | - | - | 72 | + | + |
| D | M/54 | b, a ,g,pf, npf, mo, mi, w | RC, AD | >5000 | >100 | >100 | + | - | - | 90 | 81 | - | - | 64 | ++++ | ++ |
| E | M/37 | b, a, g, w | RC, AD, AS | 747 | 58.3 | 13 | + | - | - | 81 | 90 | - | - | 42 | +++ | +++ |
| F | F/44 | b, a, g | RC, AS, OAS | 466 | 57.5 | 60.5 | + | - | - | 110 | 132 | - | - | 30 | - | - |
| G | F/36 | a g | RC | 25.7 | <0.35 | <0.35 | - | - | - | - | - | - | - | 15.5 | - | - |
| H | F/26 | no | - | <2.00 | <0.35 | <0.35 | - | - | - | - | - | - | - | 11.2 | - | - |
| I | F/32 | no | - | 2.65 | <0.35 | <0.35 | - | - | - | - | - | - | - | 10.6 | - | - |

| | | | | | | | | | | | | | | | | |
|---|---|---|---|---|---|---|---|---|---|---|---|---|---|---|---|---|
| Subjects were characterized by sex, age, and clinical diagnosis of allergies, IgE reactivity for rBet v 1 and rBet v 1 fragments (F1 and F2) was analyzed in a dot-blot assay and by means of IgE CAP. SPT, Skin prick test; b, Birch; a, animals; g, grass; pf, plant food; npf, non-plant-derived food; mo, molds; mi, mites; w, weeds; RC, rhinoconjunctivitis; AS, asthma; OAS, oral allergy syndrome | | | | | | | | | | | | | | | | |

### Example 2

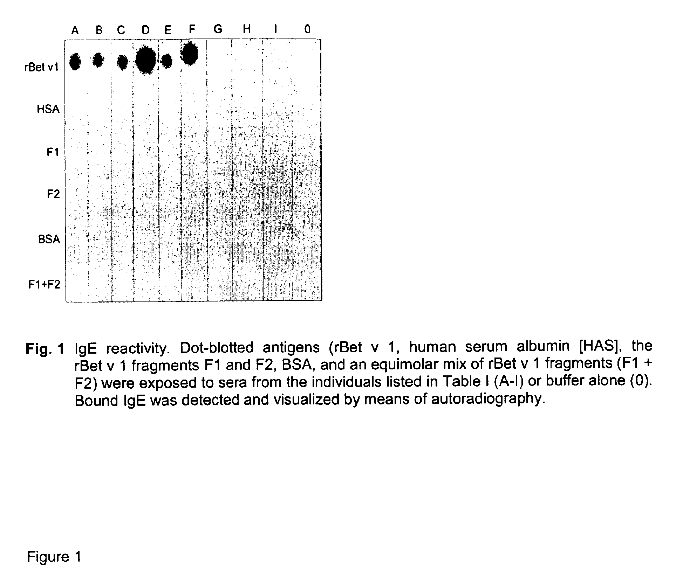

IgE reactivity to rBet v 1 and rBet v 1 fragments (F1 and F2) was tested in a nondenaturing dot-blot assay and showed that each of the 6 patients with birch pollen allergy (patients A-F) contained rBet v 1-specific IgE (Fig. 1 and Table I: 13->100 kUA/L), but none of them contained IgE specific for the Bet v 1 fragments F1 and F2 (Fig 1 and Table I). The results of the dot-blot test are shown in Figure 1.

### Example 3

Skin prick testing performed on the backs of the patients confirmed the results of the dot-blot experiments because each of the 6 patients with birch pollen allergy showed immediate-type skin reactivity rBet v 1 (20 and 40 µg/mL) after 20 minutes (Table I and see Fig. E1). None of the 6 patients with birch pollen allergy had immediate-type skin reactions to equimolar mixes of the rBet v 1 fragments, which was in agreement with the negative result from the IgE dot-blot experiment (Table I and see Fig. E1). In parallel to skin prick tests, APTs were performed with aluminium cups (Finn Chambers on Scanpor, Large, Epitest Ltd Oy) containing 160 µg of rBet v 1 or a mix containing 80 µg of each rBet v 1 fragment, as previously described (Ring et al., Int. Arch. Allergy Immunol. [1997], pp. 379-383). When the APT result was read and photo documented after 48 hours, it was found that rBet v 1 induced a positive eczematous reaction of varying intensity in each of the patients with birch pollen allergy and AD (subjects A-E) but not in the patient with birch pollen allergy without AD (subject F), the allergic patient without birch pollen allergy (subject G), and the 2 nonallergic persons (subjects H and I; Table I and see Fig. E1). The non-IgE-reactive rBet v 1 fragment mix induced an eczematous reaction in 4 of the 5 patients with birch pollen allergy and AD (subjects B-F) but not in any of the other persons (subjects G-I; Table I and see Fig. E1). Results of control APTs performed with Vaseline alone were negative in each of the 3 patients (data not shown).

The occurrence of IgE-bearing cells in the peripheral blood of the 3 patients was also studied using an anti-IgE antibody that can recognize FcεRI-bound IgE antibodies but an association between the presence of IgE-reactive cells and the presence or absence of IgE-mediated versus non-IgE-mediated APT reactions was not found.

The finding that Bet v 1 fragments containing only T-cell epitopes but no IgE epitopes could induce chronic allergic skin inflammation in 4 of the 5 patients with AD indicates that this reaction is not IgE mediated but is T cell dependent. The fact that 1 patient with AD (subject A) had an APT reaction only with IgE-reactive Bet v 1 but not with the Bet v 1 fragments which do not contain an IgE epitope and that subject D had a stronger reaction with rBet v 1 than with the fragments might be explained by the dominance of IgE-mediated allergen presentation mechnisms in these subjects, the loss of a critical T-cell epitope in the rBet v 1 fragments, or both.

### Example 4

Hypoallergenic Bet v 1 (F1 + 2) fragments induce the release of interferon gamma. PBMC from two birch pollen allergic patients (A, B) secrete high levels of interferon gamma in the culture supernatant after stimulation with hypoallergic Bet v 1 fragments whereas the naturally occurring antigen rBet v 1 does not. Contrary thereto the PBMC obtained from patient C (control) secreted a higher level of interferon gamma after contact with rBet v 1 than after contact with the hypoallergic fragments (F1 + 2).

These data demonstrate the predominance of non-IgE mediated mechanisms in the induction of T cell activation and interferon gamma secretion in these patients over IgE-mediated mechanism, because the IgE-reactive rBet v 1 failed to induce similar interferon gamma secretion. Interferon gamma is a cytokine inducing epithelial cell damage (see Reisinger et al., Mittermann et al., Akdis et al.). The results are shown in Table 2.

**Table 2: Secretion of IFN-gamma in PBMCs of birch allergic patients. PBMC from three birch pollen allergic patients (A-C) were incubated with rBet v 1, a mix of the hypoallergenic B v 1 fragments (F1+F2) or medium alone. The levels of interferon gamma secreted in the culture supernatants were measured.**

| | Patient A | Patient B | Patient C |
|---|---|---|---|
| **Sample** | **IFN** | **IFN** | **IFN** |
| Medium control | 1,58 | - | 1,81 |
| rBet v 1 | 3,16 | - | 3,53 |
| **F1+2** | 26,59 | 41,43 | 1,81 |

### Conclusion:

The tests will be done by measuring IgE vs. non-IgE-mediated effects in a patient. When there is evidence for non-IgE-mediated effects T cell targeting therapies are indicated. If IgE-mediated effects dominate the therapy should focus on IgE, e.g., administration of omalizumab (anti-IgE from Novartis) or neutralization of mast cell or basophile products by anti-histamines, anti-leukotriens etc.

The tests should have broad application in asthma and atopic dermatitis and food allergy.

In summary, the examples provide evidence for an important role of non-IgE-mediated reactions in chronic allergen-induced skin inflammation in patients with AD. The results also indicate that recombinant allergen derivatives lacking IgE epitopes but containing allergen-derived T-cell epitopes may be used as test reagents to reveal the contribution of IgE-mediated versus non-IgE-mediated mechanisms in chronic allergic inflammation. The methods of the present invention allows therefore a discrimination of these two types of diseases.

## Claims

1. In vitro method for determining the type of allergy wherein a sample
a) is reacted with an allergen comprising at least one epitope to which IgE antibodies bind and
b) the sample is further reacted with a hypoallergen derivative essentially corresponding to the allergen as used in step a) which does not contain any IgE epitope and
c) the extent of the reaction between allergen and the sample and the corresponding hypoallergen and the sample is measured.

2. Method according to claim 1 **characterized in that** the sample is obtained from a patient suffering from a chronic allergic disease.

3. Method according to claim 1 or 2 wherein the allergic disease is an allergic reaction of the late type.

4. Method according to claims 2 and 3 **characterized in that** the allergic disease is selected from the group consisting of atopic dermatitis, asthma and rhinitis.

5. Method according to any of the preceding claims **characterized in that** the reaction between allergen and sample and corresponding hypoallergen and sample is measured in an immunological test.

6. Method according to claim 5 **characterized in that** the immunological test is an enzyme-linked immunosorbent assay.

7. Method according to claim 5, **characterized in that** the immunological test is a dot blot test.

8. Method according to any of claims 1-4 **characterized in that** the reaction between allergen and the sample and corresponding hypoallergen and the sample is measured in a histamine release assay.

9. Method according to any of claims 1-4, **characterized in that** the reaction between allergen and the sample and corresponding hypoallergen and sample is measured by comparing the release of IFNγ.

10. Method according to any of claims 1-7 **characterized in that** the sample is a serum sample.

11. Test kit for determining the type of allergy which comprises an allergen comprising at least one epitope to which IgE antibodies can bind and a corresponding hypoallergen derivative which does not contain any IgE epitope.

12. Test kit according to claim 11 for use in a method according to any of claims 1 to 10.

13. Method for determining the type of allergy wherein a sample
a) is reacted with an allergen comprising at least one epitope to which IgE antibodies bind and
b) the sample is further reacted with a hypoallergen derivative essentially corresponding to the allergen as used in step a) which does not contain any IgE epitope and
c) the extent of the reaction between allergen and the sample and the corresponding hypoallergen and the sample is measured, **characterized in that** the sample is skin.
